(19) Europäisches Patentamt
European Patent Office
Office européen des brevets

(11) EP 1 866 045 B1

(12) **EUROPÄISCHE PATENTSCHRIFT**

(45) Veröffentlichungstag und Bekanntmachung des Hinweises auf die Patenterteilung:
**26.10.2011 Patentblatt 2011/43**

(21) Anmeldenummer: **06723567.1**

(22) Anmeldetag: **21.03.2006**

(51) Int Cl.:
*B01D 3/34* (2006.01) *B01D 3/36* (2006.01)
*B01D 3/40* (2006.01) *C07C 7/05* (2006.01)
*C07C 7/06* (2006.01) *C07C 7/08* (2006.01)

(86) Internationale Anmeldenummer:
**PCT/EP2006/002553**

(87) Internationale Veröffentlichungsnummer:
**WO 2006/100023 (28.09.2006 Gazette 2006/39)**

# (54) VERFAHREN ZUR DESTILLATIVEN REINIGUNG VON IONISCHEN FLÜSSIGKEITEN UND/ODER POLYMEREN

METHOD FOR THE DISTILLATIVE PURIFICATION OF IONIC LIQUIDS AND/OR POLYMERS

PROCEDE DE PURIFICATION DE LIQUIDES IONIQUES ET/OU POLYMÈRES PAR DISTILLATION

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **22.03.2005 DE 102005013030**

(43) Veröffentlichungstag der Anmeldung:
**19.12.2007 Patentblatt 2007/51**

(73) Patentinhaber: **Bayer Technology Services GmbH
51368 Leverkusen (DE)**

(72) Erfinder:
- **RUFFERT, Gerhard
51377 Leverkusen (DE)**
- **PFOHL, Oliver
51375 Leverkusen (DE)**
- **GRÜN, Marcus
Shanghai 200120 (DE)**

(74) Vertreter: **Lütjens, Henning
Bayer MaterialScience AG
Law and Patents
Patents and Licensing
Geb. Q 18
51368 Leverkusen (DE)**

(56) Entgegenhaltungen:
**DE-A1- 3 613 975 US-A- 3 689 375
US-A- 4 726 894**

- **THEODOR GREWER: "Trennung von Chlorwasserstoff und Wasser durch extraktive Destillation mit Schwefelsäure" CHEMIE INGENIEUR TECHNIK - CIT, Bd. 43, Nr. 11, Juni 1971 (1971-06), Seiten 655-658, XP002385737**
- **GARCIA VILLALUENGA J P ET AL: "A review on the separation of benzene/cyclohexane mixtures by pervaporation processes" JOURNAL OF MEMBRANE SCIENCE, ELSEVIER SCIENCE, AMSTERDAM, NL, Bd. 169, Nr. 2, Mai 2000 (2000-05), Seiten 159-174, XP004191967 ISSN: 0376-7388**
- **PATENT ABSTRACTS OF JAPAN Bd. 1997, Nr. 10, 31. Oktober 1997 (1997-10-31) & JP 09 165348 A (MITSUBISHI CHEM CORP), 24. Juni 1997 (1997-06-24)**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 866 045 B1

## Beschreibung

Die vorliegende Anmeldung betrifft ein Verfahren zur Entfernung von flüchtigen Verbindungen aus der ionischen Flüssigkeiten und/oder Polymeren mittels Rektifikation unter Verwendung eines Hilfsstoffs. Der Hilfsstoff dient dazu, unter technisch sinnvollen Bedingungen bzgl. Druck und Temperatur im Verdampfer der Kolonne einen beliebig kleinen Restgehalt der zu entfernenden flüchtigen Verbindungen im Sumpf der Kolonne erhalten zu können.

Ionische Flüssigkeiten oder flüssige Polymere, erfreuen sich in der Verfahrenstechnik größer werdender Beliebtheit, weil ihr niedriger Dampfdruck verschiedene Vorteile bietet. Sie werden gerne als Prozesslösemittel eingesetzt, da damit der Anteil der organischen Verunreinigungen in der Atmosphäre (VOC) reduziert wird, außerdem finden sie Verwendung als Hilfsmittel bei der Stofftrennung.

Zur großtechnischen Trennung von Stoffgemischen wird die mehrstufige Destillation unter Rückfluss, kurz Rektifikation, häufig eingesetzt. Kernelemente einer Rektifikationskolonne sind der Verdampfer im Sumpf, mit dem ein aufsteigender Dampfstrom erzeugt wird, und der Kondensator am Kopf, mit dem ein absteigender Flüssigkeitsstrom erzeugt wird. Aufsteigender Dampfstrom und absteigender Flüssigkeitsstrom werden an Einbauten intensiv in Kontakt miteinander gebracht. Durch den so erzeugten Gegenstrom ist es bei Verwendung von Verstärkerteil und Abtriebsteil möglich, die Zusammensetzungen der Ströme am Kopf und Sumpf der Kolonne mit Hilfe der Parameter Kolonnenhöhe und Rücklaufverhältnis in weiten Bereichen einzustellen. In der Industrie treten eine Vielzahl von Mischungen auf, die sich nicht einfach durch konventionelle Rektifikation, sondern vorzugsweise durch Extraktivrektifikation [Stichlmair, S. und Fair, J., Distillation, ISBN 0-471-25241-7, Seite 241ff oder Gmehling und Brehm, "Grundoperationen - Lehrbuch der Technischen Chemie, Band 2", Thieme Verlag, 1996] trennen lassen. Dieser Sachverhalt ist in dem ähnlichen Siedeverhalten der Gemischkomponenten begründet, d. h. in ihrer Eigenschaft, sich bei einem definierten Druck und einer definierten Temperatur im nahezu gleichen oder gleichen molaren Konzentrationsverhältnis auf die Dampf- und Flüssigphase zu verteilen. Ein in der Industrie häufig praktiziertes Vorgehen zur Trennung engsiedender - hierbei wird üblicherweise ein Trennfaktor $\alpha_{ij}$ (Verhältnis der Verteilungskoeffizienten der Komponenten i und j) kleiner 1,2 verstanden - oder azeotroper Systeme stellt die Zugabe eines selektiven Zusatzstoffes, des so genannten Entrainers, in einer Extraktivrektifikation dar. Ein geeigneter Zusatzstoff beeinflusst durch selektive Wechselwirkungen mit einer oder mehreren der Gemischkomponenten den Trennfaktor bei der Rektifikation, so dass die Auftrennung der engsiedenden oder azeotrop-siedenden Gemischkomponenten ermöglicht wird. Der Vorteil, eine schwerflüchtige Flüssigkeit als Entrainer einzusetzen, besteht - wie in DE 10136614 und EP 1372807 für ionische Flüssigkeiten oder in

DE 10160518 für hyperverzweigte Polymere beschrieben - darin, dass der geringe Dampfdruck des Entrainers eine Verunreinigung der Kopfkomponente mit dem Entrainer verhindert bzw. minimiert.

Ein zweites Verfahren, das in der Industrie zur Trennung von azeotropen oder engsiedenden Gemischen häufig Anwendung findet, ist die Flüssig-Flüssig Extraktion [Sattler, K., Thermische Trennverfahren, ISBN 3-527-28636-5, Kapitel 6]. Bei diesem Verfahren wird in ausgewählten Extraktionskolonnen der zu trennende flüssige Stoffstrom (Feed) im Gegenstrom zu einer flüssigen, selektiven Aufnehmerphase, dem Solvent, geführt. Der intensive Stoffaustausch zwischen Feed und Aufnehmerphase führt dazu, dass sich die Aufnehmerphase mit einer oder mehreren Komponenten des Feeds anreichert und als Extraktstrom die Extraktionskolonne verlässt. Der Feedstrom, welcher an den in den Extraktstrom übergetretenen Komponenten verarmt ist, wird als Raffinatstrom aus der Extraktionskolonne abgezogen. Sowohl Extraktstrom als auch Raffinatstrom können anschließend getrennten Rektifikationskolonnen zugeführt werden, in denen der jeweilige Strom in die einzelnen Komponenten aufgetrennt werden kann. Als Solvent können zweckmäßigerweise auch schwerflüchtige Flüssigkeiten eingesetzt werden, wie in DE 10160518 für hyperverzweigte Polymere beschrieben oder in "Ionic Liquids in Synthesis" (P. Wasserscheid und T. Welton, Wiley-VCH, ISBN 3-527-30515-7) für Ionische Flüssigkeiten. Statt Extraktionskolonnen sind zur Flüssig-Flüssig-Extraktion auch andere ein- oder mehrstufige Apparate im Einsatz, z.B. sogenannte Mixer-Settler.

Eine dritte Gattung in der Industrie anzutreffender Trennverfahren sind Membrantrennverfahren. Bei den Membrantrennverfahren wird ausgenutzt, dass von einem fluiden Feedstrom einige Komponenten schneller durch eine Membran transportiert werden als andere Komponenten. Auf diese Weise erhält man hinter der Membran einen Permeatstrom und analog der Flüssig-Flüssig-Extraktion einen an mindestens einer Komponente abgereicherten Retentatstrom. Hinter der Membran kann ein Solvent eingesetzt werden, um den Trenneffekt zu verbessern (so genannte Pertraktion - Permeation und Extraktion) oder um ein elektrisches Feld nutzen zu können, wie z.B. die Membranelektrophorese oder die Elektrofiltration/Elektrodialyse, wo über die Membran hinweg ein elektrisches Feld angelegt wird, um den Stofftransport durch die Membran auf die gewünschte Art und Weise selektiv zu beeinflussen (Membranverfahren, T. Melin und R. Rautenbach, Springer-Verlag, 2004). Als Solvent können zweckmäßigerweise auch schwerflüchtige Flüssigkeiten eingesetzt werden

Aus Kostengründen ist man dabei immer bestrebt, die einzusetzende Entrainermenge bei der Extraktivrektifikation bzw. die einzusetzende Solventmenge bei der Flüssig-Flüssig Extraktion oder einem Membranverfahren zu minimieren und nach Aufreinigung dem Trennprozess wieder zuzuführen.

Probleme treten auf, wenn beispielsweise in einer der Extraktivrektifikation bzw. der Flüssig-Flüssig Extraktion

oder dem Membranverfahren assoziierten Reinigungsrektifikation ein schwerflüchtiger Entrainer bzw. ein schwerflüchtiges Solvent von allen leichter flüchtigen Verunreinigungen befreit werden muss. Üblicherweise ist eine vollständige Reinigung des Entrainers bzw. des Solvents notwendig, da Reste leichtflüchtiger Verunreinigungen den Haupttrennprozess behindern. Die schwerflüchtigen Flüssigkeiten können nicht auf beliebig hohe Reinheiten aufgereinigt werden, da im Sumpf der Kolonne dann keinerlei Fluide mehr wären, die einen Dampfdruck hätten, die zu einer Verdampfung genutzt werden könnten. Da in diesem Fall im Sumpf der Kolonne nur die schwerflüchtige Komponente vorläge, müssten im Verdampfer entweder technisch nicht realisierbar hohe Temperaturen und/oder technisch nicht realisierbar niedrige Drücke eingestellt werden. Die oben genannten Patentschriften erläutern entsprechend "Regenerierung des Entrainers (...) durch eine Abtriebskolonne. Da der Dampfdruck des (reinen) Entrainers (...) und damit auch sein Partialdruck in der Mischung mit dem Sumpfprodukt gleich null sind, kann der Entrainer (...) durch reine Verdampfung nicht vollständig im Gegenstromprozess vom Sumpfprodukt befreit werden." (DE 10136614 A1, S. 3, Z. 60ff., DE 10160518 A1, S. 4, Z. 19ff., EP 1372807 B1, S. 10, Z.46ff)

Die gleiche Problemstellung ergibt sich bei einer (einstufigen) Destillation, der Verdampfung oder auch Flashen zur Befreiung der schwerflüchtigen Komponente von allen leichter flüchtigen Verunreinigungen. In Chemical & Engineering News (American Chemical Society) vom 29.4.2002, Seite 4, ist von Prof. Albrecht Salzer zu lesen, "dass IL (Ionic Liquids) nicht destilliert werden können, was ein einfacher Weg des Recyclings wäre, sondern mit organischen Lösemitteln extrahiert werden müssen".

Analoge Problemstellungen gibt es bei chemischen Reaktionen, die in schwerflüchtigen Fluiden (z.B. Ionischen Flüssigkeiten) durchgeführt werden - z.B. beschrieben in: "Ionic Liquids in Synthesis" (P. Wasserscheid und T. Welton, Wiley-VCH, ISBN 3-527-30515-7). Hier müssen verschiedene flüchtige Stoffe, insbesondere Reaktionsprodukte, aus den schwerflüchtigen Fluiden abgetrennt werden. Je besser die Abtrennung von Produkten gelingt, umso höher ist die direkte Ausbeute und umso größer ist der Umsatz bei Wiederverwendung des recyclierten Lösemittels in Gleichgewichtsreaktionen.

In den oben genannten und weiteren Veröffentlichungen (z. B. WO A 99/41752, US 2003 0085156 A), in denen schwerflüchtige Fluide als selektive Zusatzstoffe zur Stofftrennung eingesetzt werden, werden bekannte alternative Verfahren zur Befreiung der schwerflüchtigen Fluide von Verunreinigungen vorgeschlagen, ohne aber explizit auf sie einzugehen, so z.B. je nach thermophysikalischen Eigenschaften der beteiligten Stoffe:

- Strippen mit Dämpfen oder Gasen
- Extraktion mit Flüssigkeiten oder überkritischen Gasen
- Fraktionierte Kristallisation / Ausfällen einzelner Komponenten
- Elektrolyse / Elektrochemie
- Präparative Chromatographie
- Chemische Umsetzungen vor Trennungen

WO A 2001 15175 beschreibt ein Verfahren zur Reinigung von Ionischen Flüssigkeiten, in dem die zu reinigenden Flüssigkeiten bei niedrigem Druck thermisch zersetzt werden, die Zersetzungsprodukte dabei gereinigt und wieder zur Ionischen Flüssigkeit zurückreagiert werden. Es kommt dabei allerdings zu Produktverlusten. Zudem ist die Herstellung Ionischer Flüssigkeiten und damit auch deren Rückverwandlung aus den Zersetzungsprodukten extrem teuer.

Dem Fachmann ist bekannt, dass sowohl die Verwendung wieder zu entfernender und ggf. zu reinigender Hilfsstoffe (Strippen, Extraktion), das Feststoffhandling (Kristallisation, Elektrolyse, Chromatographie) und die Nutzung chemischer Reaktionen mit großem Aufwand verbunden ist, den es zu vermeiden gilt.

Der Einsatz der schwerflüchtigen Fluide ist entsprechend zurzeit dadurch eingeschränkt, dass keine industriell geeigneten Reinigungsmethoden zur Verfügung stehen.

Es stellt sich damit, ausgehend vom genannten Stand der Technik, die Aufgabe, ein Verfahren bereitzustellen, das einfach und in industriellem Maßstab die Aufreinigung schwerflüchtiger Fluide von leichter flüchtigen Komponenten ermöglicht, dabei verfahrenstechnische Vorteile bietet und kostengünstig ist.

Die Problemstellung wird hier an einem Beispiel erläutert, das bisher nicht zufriedenstellend gelöst werden konnte und das durch das erfindungsgemäße Verfahren einer Lösung zugeführt wurde. Die Darstellung beschränkt sich hier auf die wesentlichen Aspekte einer Trennung eines Stoffgemisches A+B, im Stand der Technik beschrieben in DE 10136614 A1, EP 1372807 B1 und DE 10160518 A1 (s. Fig. 1):

In der betrachteten Extraktivdestillation wird am Kopf der Kolonne ein schwerflüchtiger Entrainer E zugegeben,

in dem sich die Komponente B deutlich besser löst als die Komponente A, so dass Komponente A am Kopf der Kolonne erhalten wird und Komponente B und der Entrainer E die Kolonne zusammen am Sumpf verlassen. In einem zweiten Schritt muss nun Komponente B vom Entrainer getrennt werden, damit der Entrainer in die Kolonne zurückgeführt werden kann und reines Produkt B erhalten wird. Hierbei ist es von Interesse, dass sowohl Komponente B in hoher Reinheit gewonnen wird als auch der Entrainer. Würde der Entrainer signifikante Mengen an Komponente B enthalten, so würde bei der Rückführung an den Kopf der Kolonne die enthaltene Komponente B das Kopfprodukt (reines A) verunreinigen. Wegen des oben erwähnten großen Aufwandes bei der Auftrennung durch andere Trennmethoden wäre eine Rektifikation zur Trennung von Entrainer und Komponente B in dem zweiten Schritt wünschenswert oder im Interesse eines wirtschaftlichen Verfahrens sogar notwendig. Die in DE 10136614 A1, S. 3, Z. 60ff., DE 10160518 A1, S. 4, Z. 19ff, EP 1372807 B1, S. 10, Z. 46 ff. oder von Prof. Albrecht Salzer, Chemical & Engineering News vom 29.4.2002, Seite 4, genannten Gründe scheinen diesen Einsatz jedoch zu verhindern.

Gegenstand der vorliegenden Erfindung ist daher ein Verfahren gemäß Anspruch 1.

Im erfindungsgemäßen Verfahren, steuert der dort verwendete Hilfsstoff durch seinen Partialdruck im Sumpf der Kolonne einen Anteil von mindestens 50 %, bevorzugt mindestens 75 %, besonders bevorzugt mindestens 90 % bei, so dass die Befreiung der ionischen Flüssigkeiten und/oder des Polymers mittels Rektifikation bei technisch sinnvollen Bedingungen von allen zu entfernenden flüchtigen Verunreinigungen (mit Ausnahme des Hilfsstoffs) möglich wird und das oben und in DE 10136614 A1 und DE 10160518 A1 und EP 1372807 B1 beschriebene Problem der beschränkten Aufreinigung auf überraschend einfache Art und Weise gelöst werden kann.

Im Sumpf der Destillationseinheit ist der Partialdruck des Hilfsstoffes bzw. des Hilfsstoffgemisches dabei höher als der dortige Partialdruck der über Kopf als Verunreinigungen zu entfernenden Komponenten.

Der Druck im Sumpf der Kolonne abzüglich des dort herrschenden Partialdrucks des Hilfsstoffes bzw. des Hilfsstoffgemisches beträgt im erfindungsgemäßen Verfahren höchstens 10 mbar, bevorzugt höchstens 5 mbar, besonders bevorzugt höchstens 2 mbar.

Der Hilfsstoff bzw. das Hilfsstoffgemisch soll erfindungsgemäß die Reinigungskolonne zusammen mit der ionischen Flüssigkeiten und/oder dem Polymer größtenteils am Sumpf der Reinigungskolonne verlassen, wobei unter größtenteils mindestens 50 Gew.-%, bevorzugt mindestens 75 Gew.-%, besonders bevorzugt mindestens 95 Gew.-% verstanden wird. Der Großteil der leichter flüchtigen Verunreinigungen verlässt die Reinigungskolonne am Kopf.

Die verwendete Menge des Hilfsstoffes, einzeln oder in Mischung, richtet sich nach wirtschaftlichen und thermodynamischen Gesichtspunkten und nach der Art der Anwendung; bezogen auf den Sumpfstrom der Reinigungskolonne können es in einer bevorzugten Ausführung des erfindungsgemäßen Verfahrens bei einer möglichst vollständigen Reinigung der ionischen Flüssigkeiten und/oder des Polymers weniger als 40 Gew.-%, bevorzugt weniger als 20 Gew.-%, ganz besonders bevorzugt weniger als 10 Gew.-% sein.

In einer weiteren Ausführungsform bei beispielsweise einer an eine chemische Reaktion angegliederten Aufreinigung der ionischen Flüssigkeiten und/oder des Polymers mit Edukt als Hilfsstoff kann die Hilfsstoffmenge beliebig groß sein, da bei Rückführung der gereinigten ionischen Flüssigkeiten und/oder des gereinigten Polymers das Edukt wieder Eingang in die Reaktion fände.

Der Reinstoffdampfdruck des Hilfsstoffes bzw. des Hilfsstoffgemisches ist bei Betriebstemperatur deutlich größer als der der ionischen Flüssigkeiten und/oder des Polymers und zwar um einen Faktor von mindestens 10, bevorzugt um einen Faktor von mindestens 100, besonders bevorzugt um einen Faktor von mindestens 1000, wobei der Reinstoffdampfdruck der ionischen Flüssigkeiten und/oder des Polymers bei Sumpftemperatur in der Regel unter 5 mbar liegt. Hilfsstoffe, die oberhalb ihrer kritischen Temperatur eingesetzt werden, haben - sinngemäß - bei ihrer kritischen Temperatur bereits einen höheren Dampfdruck als die ionische Flüssigkeit und/oder das Polymer.

Ohne die allgemeine Verwendbarkeit der Erfindung hierauf beschränken zu wollen, wird die Lösung des beispielhaft beschriebenen Problems durch das erfindungsgemäße Verfahren erläutert (s. Fig. 2).

Bei der beschriebenen Extraktivdestillation wird erfindungsgemäß zusammen mit dem Entrainer E ein Hilfsstoff H im Kreis gefahren, der die Extraktivdestillationskolonne zusammen mit dem Entrainer E und Produkt B im Sumpf verlässt und der in einer zweiten Kolonne, der Reinigungskolonne, die Abtrennung des Produktes B ermöglicht, das die Reinigungskolonne am Kopf verlässt, und dann zusammen mit dem Entrainer am Sumpf austritt. Entrainer und Hilfsstoff werden dann wieder am Kopf der Extraktivdestillationskolonne zugegeben. Über die Konstruktion der Reinigungskolonne, speziell Kolonnenhöhe und Rücklaufverhältnis, lässt sich dann die Menge der Verunreinigung B im rückgeführten Entrainer beliebig absenken ohne die oben erläuterten Probleme aufzuweisen.

Das erfindungsgemäße Verfahren bietet die Vorteile, dass bei geeigneter Wahl des Hilfsstoffes derselbe nur in kleinen Konzentrationen dosiert werden muss und er dennoch technisch sinnvolle Bedingungen (hoher Druck, niedrige Temperatur) im Verdampfer ermöglicht, dass er in der Reinigungskolonne gut vom Kopfprodukt getrennt werden kann, dass - im Falle, dass das erfindungsgemäße Aufreinigungsverfahren mit einem Trennverfahren assoziiert ist - er später nicht von der ionischen Flüssigkeiten und/oder dem Polymer abgetrennt werden muss, weil er in den verwendeten Konzentrationen im restlichen Prozess nicht signifikant stört, dass er, bei entsprechender Aufgabenstellung, im Kreis gefahren werden kann, so dass sich die Kosten für den Hilfsstoff reduzieren und dass er, bei entsprechender Aufgabenstellung, zusammen mit der ionischen Flüssigkeiten und/oder dem Polymer im Kreis gefahren werden kann, um mehrere der oben genannten Vorteile vereinen zu können.

Vom klassischen Verfahren einer Wasserdampfdestillation, bei der durch Zugabe des Hilfsstoffes Wasser eine niedrige Siedetemperatur erzielt wird, setzt sich das erfindungsgemäße Verfahren offensichtlich dadurch ab, dass der hier zugegebene Hilfsstoff nur in geringen Mengen eingesetzt wird und er die Destillationseinheit, in der er als Hilfsstoff wirkt, praktisch ausschließlich durch den Sumpf verlässt. Da die Grundlage der Wasserdampfdestillation die Unmischbarkeit des Wassers mit den zu verdampfenden Komponenten ist, um den Gesamtdruck zu erhöhen, ist es überdies abwegig, von der Wasserdampfdestillation auf die hier gemachte Erfindung zu schließen, da letztere ja nicht auf diesen Effekt angewiesen ist.

Vom klassischen Verfahren einer Strippung, bei der durch Zugabe eines Hilfsstoffes als Gas oder Dampf eine Dampfphase in der Destillation erzeugt wird, setzt sich das erfindungsgemäße Verfahren offensichtlich dadurch ab, dass der hier zugegebene Hilfsstoff die Destillation weitgehend am Sumpf verlässt, während beim Strippen der Hilfsstoff die Destillation weitgehend am Kopf verlässt. Da die Grundlage des Strippens gerade ist, dass der Hilfsstoff die Destillation weitgehend am Kopf verlässt, ist es überdies abwegig, von der Strippung auf die hier gemachte Erfindung zu schließen, da letztere diesbezüglich quasi das Gegenteil darstellt.

Das erfindungsgemäße destillative Reingungsverfahren setzt sich von den Extraktivdestillationen dadurch ab, dass bei einer postulierten chemischen und thermischen Stabilität der Komponenten bei ähnlicher Zahl theoretischen Stufen eine ähnlich vollständige Abtrennung der flüchtigen Komponenten von der ionischen Flüssigkeiten und/oder dem Polymer ohne Hilfsstoff erreicht werden könnte, wenn der Betriebsdruck abgesenkt oder die Betriebstemperatur angehoben würde, d.h. dadurch dass ein Entrainer bei der Extraktivdestillation bestimmungsgemäß die relative Flüchtigkeit der zu trennenden Komponenten signifikant ändert, was bei dem Einsatz des Hilfsstoffes in der erfindungsgemäßen Destillation nicht nötig ist.

Bei den nach dem erfindungsgemäßen Verfahren zu reinigenden ionischen Flüssigkeiten und/oder des Polymeren handelt es sich um reine chemische Verbindungen oder Mischungen, die bei Betriebstemperatur des Reinigungsprozesses Dampfdrücke unter 10 mbar, bevorzugt unter 1 mbar, besonders bevorzugt unter 0,1 mbar aufweisen. Die Betriebstemperatur ist üblicher Weise eine Temperatur, bei der keine störenden Zersetzungsreaktionen o.ä. auftreten; dieses wird in der Regel eine Temperatur unterhalb von 600°C, bevorzugt unterhalb von 350°C, besonders bevorzugt unterhalb von 200°C sein.

Ionische Flüssigkeiten sind insbesondere solche, wie sie von P. Wasserscheid und W. Keim in der Angewandten Chemie, 2000, 112, 3926-3945, definiert sind. Sie können einzeln oder in Mischungen mit einem oder mehreren Polymeren eingesetzt werden. Ionische Flüssigkeiten sind im Vergleich zu herkömmlichen Salzen bei wesentlich geringeren Temperaturen (in der Regel unterhalb von 200°C) flüssig und besitzen häufig einen Schmelzpunkt unterhalb von 0°C, im Einzelfall bis -96°C, was wichtig für beispielsweise die industrielle Umsetzung der Extraktivrektifikation ist. Darüber hinaus sind ionische Flüssigkeiten in der Regel nicht brennbar, nicht korrosiv und wenig viskos und zeichnen sich in der Regel durch einen geringen, zum Teil zurzeit nicht messbaren, Dampfdruck aus. Sie besitzen häufig sehr gute Löslichkeiten für eine große Anzahl organischer, anorganischer und polymerer Substanzen.

Bevorzugt werden Ionische Flüssigkeiten betrachtet, die - oder deren Ionen - in einer oder mehreren der folgenden Publikationen oder dort zitierten Literaturstellen genannt werden: EP 1372807 B1, DE 10154052 A1, DE 10206808 A1, WO 03/037835 A3, WO A 2002 074718, US 6339182 B1, US 4359596 B, US 5220106 B, DE 19901524 A1, WO A 00/16902, WO A1 01/98239, WO A1 03/051894, WO A1 03/062251, DE A1 19919494, "Ionic Liquids in Synthesis" ISBN 3527305157, "Molten Salt Techniques" ISBN 0306435543, "Ionic Liquids" ISBN 0841237891, "Ionic Liquids as Green Solvents" ISBN 0841238561, "Green Industrial Applications of Ionic Liquids" ISBN 1402011377, Chem. Rev. 1999, 99, 2071-2083.

Als ionische Flüssigkeiten werden erfindungsgemäß solche Verbindungen bezeichnet, die mindestens eine positive und mindestens eine negative Ladung aufweisen, insgesamt jedoch ladungsneutral sind, und einen Schmelzpunkt unter 200°C aufweisen, bevorzugt unter 100, besonders bevorzugt unter 50°C.

Die Ionischen Flüssigkeiten können auch mehrere positive oder negative Ladungen aufweisen, beispielsweise 1 bis 5, bevorzugt 1 bis 4, besonders bevorzugt 1 bis 3, ganz besonders bevorzugt 1 bis 2, insbesondere jedoch je eine positive und negative Ladung. Die Ladungen können sich an verschiedenen lokalisierten oder delokalisierten Bereichen innerhalb eines Moleküls befinden, also betainartig, oder auf je ein getrenntes Anion und Kation verteilt sein. Bevorzugt sind solche Ionischen Flüssigkeiten, die aus mindestens einem Kation und mindestens einem Anion aufgebaut sind. Kation und Anion können, wie oben ausgeführt, ein oder mehrfach, bevorzugt einfach geladen sein.

Bevorzugt als Kation sind Ammonium- oder Phosphoniumionen, oder solche Kationen, die mindestens einen fünf- bis sechsgliedrigen Heterocyclus enthalten, der mindestens ein Phosphor- oder Stickstoffatom sowie gegebenenfalls ein Sauerstoff- oder Schwefelatom aufweist, besonders bevorzugt solche Verbindungen, die mindestens einen fünf- bis sechsgliedrigen Heterocyclus enthalten, der ein, zwei oder drei Stickstoffatome und ein Schwefel- oder ein Sauerstoffatom aufweist, ganz besonders bevorzugt solche mit ein oder zwei Stickstoffatomen.

Besonders bevorzugte Ionische Flüssigkeiten sind solche, die ein Molgewicht unter 1000 g/mol aufweisen,

ganz besonders bevorzugt unter 350 g/mol.

Weiterhin sind solche Kationen bevorzugt, die ausgewählt sind aus den Verbindungen der Formeln 1 bis 23

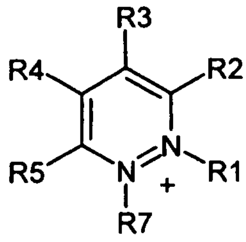
1

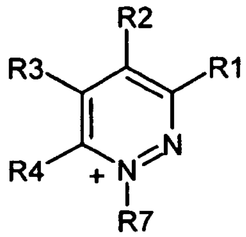
2

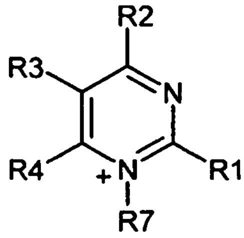
3

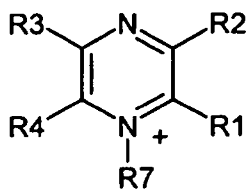
4

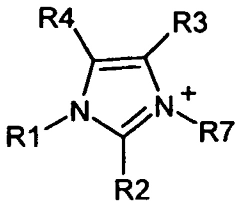
5

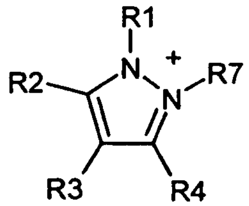
6

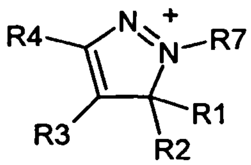
7

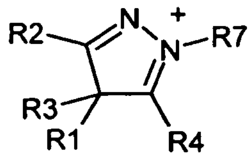
8

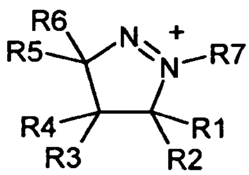
9

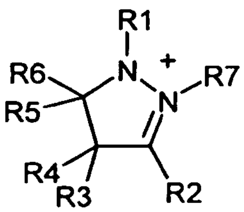
10

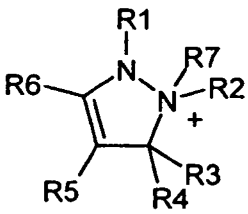
11

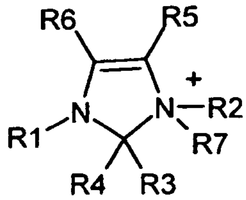
12

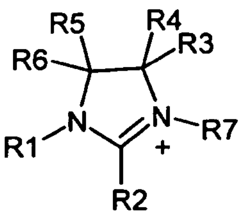
13

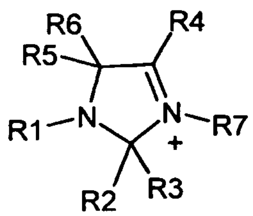
14

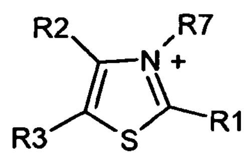
15

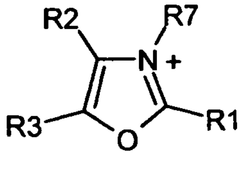
16

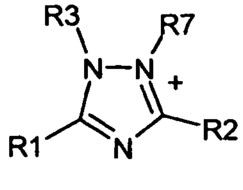
17

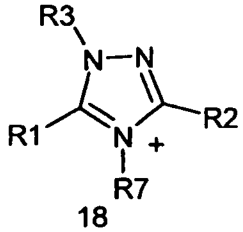
18

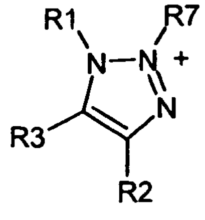
19

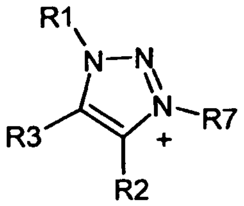
20

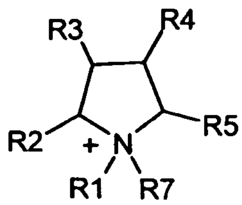
21

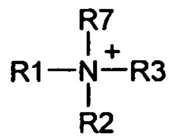
22

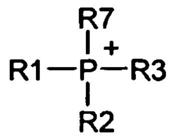
23

sowie Oligo-bzw. Polymere, die diese Strukturen enthalten, worin

$R^1$, $R^2$, $R^3$, $R^4$, $R^5$, $R^6$ und $R^7$ unabhängig voneinander jeweils $C_1$ - $C_{18}$-Alkyl, gegebenenfalls durch ein oder mehrere Sauerstoff- und/oder Schwefelatome und/oder ein oder mehrere substituierte oder unsubstituierte Iminogruppen unterbrochenes $C_2$ - $C_{18}$-Alkyl, $C_6$ - $C_{12}$-Aryl, $C_5$ - $C_{12}$-Cycloalkyl oder einen fünf- bis sechsgliedrigen, Sauerstoff- , Stickstoff- und/oder Schwefelatome aufweisenden Heterocyclus bedeuten oder zwei von ihnen gemeinsam einen ungesättigten, gesättigten oder aromatischen und gegebenenfalls durch ein oder mehrere Sauerstoff- und/oder Schwefelatome und/oder ein oder mehrere substituierte oder unsubstituierte Iminogruppen unterbrochenen Ring bilden, wobei die genannten Reste jeweils durch funktionelle Gruppen, Aryl, Alkyl, Aryloxy, Alkyloxy, Halogen, Heteroatome und/oder Heterocyclen substituiert sein können.

$R^1$, $R^2$, $R^3$, $R^4$, $R^5$ und $R^6$ können zusätzlich dazu Wasserstoff bedeuten.

$R^7$ kann darüberhinaus $C_1$ - $C_{18}$ -Alkyloyl (Alkylcarbonyl), $C_1$ - $C_{18}$- Alkyloxycarbonyl, $C_5$-$C_{12}$-Cycloalkylcarbonyl oder $C_6$ - $C_{12}$-Aryloyl (Arylcarbonyl) bedeuten, wobei die genannten Reste jeweils durch funktionelle Gruppen, Aryl, Alkyl, Aryloxy, Alkyloxy, Halogen, Heteroatome und/oder Heterocyclen substituiert sein können.

Gegebenenfalls durch funktionelle Gruppen, Aryl, Alkyl, Aryloxy, Alkyloxy, Halogen, Heteroatome und/oder Heterocyclen substituiertes $C_1$ - $C_{18}$-Alkyl steht dabei beispielsweise für Methyl, Ethyl, Propyl, Isopropyl, n-Butyl, sec-Butyl, tert.-Butyl, Pentyl, Hexyl, Heptyl, Octyl, 2-Ethylhexyl, 2,4,4-Trimethylpentyl, Decyl, Dodecyl, Tetradecyl, Hetadecyl, Octadecyl, 1,1-Dimethylpropyl, 1,1-Dimethylbutyl, 1,1,3,3-Tetramethylbutyl, Benzyl, 1-Phenylethyl, 2-Phenylethyl, $\alpha,\alpha$-Dimethylbenryl, Benzhydryl, p-Tolylmethyl,1-(p-Butylphenyl)ethyl, p-Chlorbenzyl, 2,4-Dichlorbenzyl, p-Methoxybenzyl, m-Ethoxybenzyl, 2-Cyanoethyl, 2-Cyanopropyl, 2-Methoxycarbonethyl, 2-Ethoxycarbonylethyl, 2-Butoxycarbonylpropyl, 1,2-Di-(methoxycarbonyl)-ethyl, 2-Methoxyethyl, 2-Ethoxyethyl, 2-Butoxyethyl, Diethoxymethyl, Diethoxyethyl, 1,3-Dioxan-2-yl, 2-Methyl-1,3-dioxolan-2-yl, 4-Methyl-1,3-dioxolan-2-yl, 2-Isopropoxyethyl, 2-Butoxypropyl, 2-Octyloxyethyl, Chlormethyl, 2-Chlorethyl, Trichlormethyl, Trifluormethyl, 1,1-Dimethyl-2-chlorethyl, 2-Methoxyisopropyl, 2-Ethoxyethyl, Butylthiomethyl, 2-Dodecylthioethyl, 2-Phenylthioethyl, 2,2,2-Trifluorethyl, 2-Hydroxyethyl, 2-Hydroxypropyl, 3-Hydroxypropyl, 4-Hydroxybutyl, 6-Hydroxyhexyl, 2-Aminoethyl, 2-Aminopropyl, 3-Aminopropyl, 4-Aminobutyl, 6-Aminohexyl, 2-Methylaminoethyl, 2-Methylaminopropyl, 3-Methylaminopropyl, 4-Methylaminobutyl, 6-Methylaminohexyl, 2-Dimethylaminoethyl, 2-Dimethylaminopropyl, 3-Dimethylaminopropyl, 4-Dimethylaminobutyl, 6-Dimethylaminohexyl, 2-Hydroxy-2,2-dimethylethyl, 2-Phenoxyethyl, 2-Phenoxypropyl, 3-Phenoxypropyl, 4-Phenoxybutyl, 6-Phenoxyhexyl, 2-Methoxyethyl, 2-Methoxypropyl, 3-Methoxypropyl, 4-Methoxybutyl, 6- Methoxyhexyl, 2-Ethoxyethyl, 2-Ethoxypropyl, 3-Ethoxypropyl, 4-Ethoxybutyl oder 6-Ethoxyhexyl und

gegebenenfalls durch ein oder mehrere Sauerstoff- und/oder Schwefelatome und/oder ein oder mehrere substituierte oder unsubstituierte Iminogruppen unterbrochenes $C_2$ - $C_{18}$ -Alkyl beispielsweise für 5-Hydroxy-3-oxa-pentyl, 8-Hydroxy-3,6-dioxa-octyl, 11-Hydroxy-3,6,9-trioxaundecyl, 7-Hydroxy-4-oxa-heptyl, 11-Hydroxy-4,8-dioxa-undecyl, 15-Hydroxy-4,8,12-trioxapentadecyl, 9-Hydroxy-5-oxa-nonyl, 14-Hydroxy-5,10-oxa-tetradecyl, 5-Methoxy-3-oxa-pentyl, 8-Methoxy-3,6-dioxa-octyl, 11-Methoxy-3,6,9-trioxa-undecyl, 7-Methoxy-4-oxa-heptyl, 11-Methoxy-4,8-dioxa-undecyl, 15-Methoxy-4,8,12-trioxa-pentadecyl, 9-Methoxy-5-oxanonyl, 14-Methoxy-5,10-oxa-tetradecyl, 5-Ethoxy-3-oxa-pentyl, 8-Ethoxy-3,6-dioxa-octyl, 11-Ethoxy-3,6,9-trioxa-undecyl, 7-Ethoxy-4-oxa-heptyl, 11-Ethoxy-4,8-dioxa-undecyl, 15-Ethoxy-4,8,12-trioxapentadecyl, 9-Ethoxy-5-oxa-nonyl oder 14-Ethoxy-5,10-oxa-tetradecyl.

Bilden zwei Reste einen Ring, so können diese Reste gemeinsam bedeuten 1,3-Propylen, 1,4-Butylen, 2-Oxa-1,3-propylen, 1-Oxa-1,3-propylen, 2-Oxa-1,3-propylen, 1-Oxa-1,3-propenylen, 1-Aza-1,3- propenylen, 1-$C_1$-$C_4$-Alkyl-1-aza-1,3-propenylen, 1,4-Buta-1,3-dienylen, 1-Aza-1,4-buta-1,3-dienylen oder 2- Aza-1,4-buta-1,3-dienylen.

Die Anzahl der Sauerstoff- und/oder Schwefelatome und/oder Iminogruppen ist nicht beschränkt. In der Regel

beträgt sie nicht mehr als 5 in dem Rest, bevorzugt nicht mehr als 4 und ganz besonders bevorzugt nicht mehr als 3.

Weiterhin befinden sich zwischen zwei Heteroatomen in der Regel mindestens ein Kohlenstoffatom, bevorzugt mindestens zwei.

Substituierte und unsubstituierte Iminogruppen können beispielsweise Imino-, Methylimino-, *iso*-Propylimino, n-Butylimino oder tert-Butylimino sein.

Weiterhin steht

· funktionelle Gruppen beispielsweise für Carboxy, Carboxamid, Hydroxy, Di-($C_1$-$C_4$-Alkyl)-amino, $C_1$-$C_4$- Alkyloxycarbonyl, Cyano oder $C_1$-$C_4$ -Alkyloxy,

· gegebenenfalls durch funktionelle Gruppen, Aryl, Alkyl, Aryloxy, Alkyloxy, Halogen, Heteroatome und/ oder Heterocyclen substituiertes $C_6$ - $C_{12}$-Aryl steht beispielsweise für Phenyl, Tolyl, Xylyl, α-Naphthyl, β-Naphthyl, 4-Diphenylyl, Chlorphenyl, Dichlorphenyl, Trichlorphenyl, Difluorphenyl, Methylphenyl, Dimethylphenyl, Trimethylphenyl, Ethylphenyl, Diethylphenyl, *iso*Propylphenyl, tert.-Butylphenyl, Dodecylphenyl, Methoxyphenyl, Dimethoxyphenyl, Ethoxyphenyl, Hexyloxyphenyl, Methylnaphthyl, Isopropylnaphthyl, Chlornaphthyl, Ethoxynaphthyl, 2,6-Dimethylphenyl, 2,4,6-Trimethylphenyl, 2,6-Dimethoxyphenyl, 2,6-Dichlorphenyl, 4-Bromphenyl, 2- oder 4-Nitrophenyl, 2,4- oder 2,6-Dinitrophenyl, 4-Dimethylaminophenyl, 4-Acetylphenyl, Methoxyethylphenyl oder Ethoxymethylphenyl,

- gegebenenfalls durch funktionelle Gruppen, Aryl, Alkyl, Aryloxy, Alkyloxy, Halogen, Heteroatome und/oder Heterocyclen substituiertes $C_5$ - $C_{12}$-Cycloalkyl steht beispielsweise für Cyclopentyl, Cyclohexyl, Cyclooctyl, Cyclododecyl, Methylcyclopentyl, Dimethylcyclopentyl, Methylcyclohexyl, Dimethylcyclohexyl, Diethylcyclohexyl, Butylcyclohexyl, Methoxycyclohexyl, Dimethoxycyclohexyl, Diethoxycyclohexyl, Butylthiocyclohexyl, Chlorcyclohexyl, Dichlorcyclohexyl, Dichlorcyclopentyl sowie ein gesättigtes oder ungesättigtes bicyclisches System wie z.B. Norbornyl oder Norbornenyl,

· ein fünf- bis sechsgliedriger, Sauerstoff- , Stickstoff- und/oder Schwefelatome aufweisender Heterocyclus steht beispielsweise für Furyl, Thiophenyl, Pyrryl, Pyridyl, Indolyl, Benzoxazolyl, Dioxolyl, Dioxyl, Benzimidazolyl, Benzthiazolyl, Dimethylpyridyl, Methylchinolyl, Dimethylpyrryl, Methoxyfuryl, Dimethoxypyridyl, Difluorpyridyl, Methylthiophenyl, Isopropylthiophenyl oder tert.-Butylthiophenyl und

· $C_1$ bis $C_4$-Alkyl steht beispielsweise für Methyl, Ethyl, Propyl, Isopropyl, n-Butyl, sec-Butyl oder tert.-Butyl.

$C_1$ - $C_{18}$ -Alkyloyl (Alkylcarbonyl) steht beispielsweise für Acetyl, Propionyl, n-Butyloyl, sec-Butyloyl, tert.-Butyloyl, 2-Ethylhexylcarbonyl, Decanoyl, Dodecanoyl, Chloracetyl, Trichloracetyl oder Trifluoracetyl.

$C_1$ - $C_{18}$ - Alkyloxycarbonyl steht beispielsweise für Methyloxycarbonyl, Ethyloxycarbonyl, Propyloxycarbonyl, Isopropyloxycarbonyl, n-Butyloxycarbonyl, sec-Butyloxycarbonyl, tert.-Butyloxycarbonyl, Hexyloxycarbonyl, 2-Ethylhexyloxycarbonyl oder Benzyloxycarbonyl.

$C_5$ - $C_{12}$ - Cycloalkylcarbonyl steht beispielsweise für Cyclopentylcarbonyl, Cyclohexylcarbonyl oder Cyclododecylcarbonyl.

$C_6$ - $C_{12}$ -Aryloyl (Arylcarbonyl) steht beispielsweise für Benzoyl, Toluyl, Xyloyl, α-Naphthoyl, β-Naphthoyl, Chlorbenzoyl, Dichlorbenzoyl, Trichlorbenzoyl oder Trimethylbenzoyl.

Bevorzugt stehen $R^1$, $R^2$, $R^3$, $R^4$, $R^5$ und $R^6$ unabhängig voneinander für Wasserstoff, Methyl, Ethyl, n-Butyl, 2-Hydroxyethyl, 2-Cyanoethyl, 2-(Methoxycarbonyl)-ethyl, 2-(Ethoxycarbonyl)-ethyl, 2-(n-Butoxycarbonyl)-ethyl, Dimethylamino, Diethylamino und Chlor.

Bevorzugt steht $R^7$ für Methyl, Ethyl, n-Butyl, 2-Hydroxyethyl, 2-Cyanoethyl, 2-(Methoxycarbonyl)-ethyl, 2-(Ethoxycarbonyl)-ethyl, 2-(n-Butoxycarbonyl)-ethyl, Acetyl, Propionyl, t-Butyryl, Methoxycarbonyl, Ethoxycarbonyl oder n-Butoxycarbonyl.

Besonders bevorzugte Pyridiniumionen entsprechend Formel 1 sind solche, bei denen mindestens einer der Reste $R^1$ bis $R^5$ für Methyl, Ethyl oder Chlor ist, $R^7$ Acetyl, Methyl, Ethyl oder n-Butyl und alle anderen für Wasserstoff stehen, oder $R^3$ für Dimethylamino, $R^7$ Acetyl, Methyl, Ethyl oder n-Butyl und alle anderen Wasserstoff sind oder $R^7$ Acetyl, Methyl, Ethyl oder n-Butyl und alle anderen für Wasserstoff stehen oder $R^2$ für Carboxy oder Carboxamid, (und?) $R^7$ für Acetyl, Methyl, Ethyl oder n-Butyl und alle anderen für Wasserstoff stehen oder $R^1$ und $R^2$ oder $R^2$ und $R^3$ für 1,4-Buta-1,3-dienylen, $R^7$ Acetyl, Methyl, Ethyl oder n-Butyl und alle anderen für Wasserstoff stehen.

Besonders bevorzugte Pyridaziniumionen entsprechend Formel 2 sind solche, bei denen einer der Reste $R^1$ bis $R^4$ für Methyl oder Ethyl, $R^7$ Acetyl, Methyl, Ethyl oder n-Butyl und alle anderen für Wasserstoff stehe oder $R^7$ für Acetyl, Methyl, Ethyl oder n-Butyl, und alle anderen für Wasserstoff stehen.

Besonders bevorzugte Pyrimidiniumionen entsprechend Formel 3 sind solche, bei denen $R^2$ bis $R^4$ für Wasserstoff oder Methyl stehen, $R^7$ für Acetyl, Methyl, Ethyl oder n-Butyl und $R^1$ für Wasserstoff, Methyl oder Ethyl steht,

oder $R^2$ und $R^4$ für Methyl, $R^3$ für Wasserstoff und $R^1$ für Wasserstoff, Methyl oder Ethyl und $R^7$ für Acetyl, Methyl, Ethyl oder n-Butyl steht.

Besonders bevorzugte Pyraziniumionen entsprechend Formel 4 sind solche, bei denen

$R^1$ bis $R^4$ alle für Methyl und

$R^7$ für Acetyl, Methyl, Ethyl oder n-Butyl oder $R^7$ für Acetyl, Methyl, Ethyl oder n-Butyl und alle anderen für Wasserstoff stehen.

Besonders bevorzugte Imidazoliumionen entsprechend Formel 5 sind solche, bei denen unabhängig voneinander

$R^1$ ausgewählt ist aus der Gruppe bestehend aus Methyl, Ethyl, n-Propyl, n- Butyl, n-Pentyl, n-Octyl, n-Decyl, n-Dodecyl, 2-Hydroxyethyl oder 2-Cyanoethyl,

$R^7$ für Acetyl, Methyl, Ethyl oder n-Butyl und.

$R^2$ bis $R^4$ unabhängig voneinander für Wasserstoff, Methyl oder Ethyl stehen.

Besonders bevorzugte 1H-Pyrazoliumionen entsprechend Formel 6 sind solche, bei denen unabhängig voneinander

$R^1$ für Wasserstoff, Methyl oder Ethyl,

$R^2$, $R^3$ und $R^4$ für Wasserstoff oder Methyl und

$R^7$ für Acetyl, Methyl, Ethyl oder n-Butyl stehen.

Besonders bevorzugte 3H-Pyrazoliumionen entsprechend Formel 7 sind solche, bei denen unabhängig voneinander

$R^1$ für Wasserstoff, Methyl oder Ethyl,

$R^2$, $R^3$ und $R^4$ für Wasserstoff oder Methyl und

$R^7$ für Acetyl, Methyl, Ethyl oder n-Butyl stehen.

Besonders bevorzugte 4H-Pyrazoliumionen entsprechend Formel 8 sind solche, bei denen unabhängig voneinander

$R^1$ bis $R^4$ für Wasserstoff oder Methyl und

$R^7$ für Acetyl, Methyl, Ethyl oder n-Butyl stehen.

Besonders bevorzugte 1-Pyrazoliniumionen entsprechend Formel 9 sind solche, bei denen unabhängig voneinander

$R^1$ bis $R^6$ für Wasserstoff oder Methyl und

$R^7$ für Acetyl, Methyl, Ethyl oder n-Butyl stehen.

Besonders bevorzugte 2-Pyrazoliniumionen entsprechend Formel 10 sind solche, bei denen unabhängig voneinander

$R^1$ für Wasserstoff, Methyl, Ethyl oder Phenyl,

$R^7$ für Acetyl, Methyl, Ethyl oder n-Butyl und

$R^2$ bis $R^6$ für Wasserstoff oder Methyl stehen.

Besonders bevorzugte 3-Pyrazoliniumionen entsprechend Formel 11 sind solche, bei denen unabhängig voneinander

$R^1$ oder $R^2$ für Wasserstoff, Methyl, Ethyl oder Phenyl,

$R^7$ für Acetyl, Methyl, Ethyl oder n-Butyl und

$R^3$ bis $R^6$ für Wasserstoff oder Methyl

stehen.

Besonders bevorzugte Imidazoliniumionen entsprechend Formel 12 sind solche, bei denen unabhängig voneinander

$R^1$ oder $R^2$ für Wasserstoff, Methyl, Ethyl, n-Butyl oder Phenyl,

$R^7$ für Acetyl, Methyl, Ethyl oder n-Butyl und

$R^3$ oder $R^4$ für Wasserstoff, Methyl oder Ethyl und

$R^5$ oder $R^6$ für Wasserstoff oder Methyl

stehen.

Besonders bevorzugte Imidazoliniumionen entsprechend Formel 13 sind solche, bei denen unabhängig voneinander

$R^1$ oder $R^2$ für Wasserstoff, Methyl oder Ethyl,

$R^7$ für Acetyl, Methyl, Ethyl oder n-Butyl und

$R^3$ bis $R^6$ für Wasserstoff oder Methyl

stehen.

Besonders bevorzugte Imidazoliniumionen entsprechend Formel 14 sind solche, bei denen unabhängig voneinander

$R^1$, $R^2$ oder $R^3$ für Wasserstoff, Methyl oder Ethyl,

$R^7$ für Acetyl, Methyl, Ethyl oder n-Butyl und

$R^4$ bis $R^6$ für Wasserstoff oder Methyl

stehen.

Besonders bevorzugte Thiazoliumionen entsprechend Formel 15 oder Oxazoliumionen entsprechend Formel 16 sind solche, bei denen unabhängig voneinander

$R^1$ für Wasserstoff, Methyl, Ethyl oder Phenyl,

$R^7$ für Acetyl, Methyl, Ethyl oder n-Butyl und

$R^2$ oder $R^3$ für Wasserstoff oder Methyl

stehen.

Besonders bevorzugte 1,2,4- Triazoliumionen entsprechend Formel 17 und 18 sind solche, bei denen unabhängig voneinander

$R^1$ oder $R^2$ für Wasserstoff, Methyl, Ethyl oder Phenyl,

$R^7$ für Acetyl, Methyl, Ethyl oder n-Butyl und

$R^3$ für Wasserstoff, Methyl oder Phenyl

stehen.

Besonders bevorzugte 1,2,3- Triazoliumionen entsprechend Formel 19 und 20 sind solche, bei denen unabhängig voneinander

$R^1$ für Wasserstoff, Methyl oder Ethyl,

$R^7$ für Acetyl, Methyl, Ethyl oder n-Butyl und

$R^2$ oder $R^3$ für Wasserstoff oder Methyl stehen oder

$R^2$ und $R^3$ für 1,4-Buta-1,3-dienylen und alle anderen für Wasserstoff stehen.

Besonders bevorzugte Pyrrolidiniumionen entsprechend Formel 21 sind solche, bei denen unabhängig voneinander

$R^1$ und $R^7$ für Acetyl, Methyl, Ethyl oder n-Butyl stehen und

$R^2$, $R^3$, $R^4$ und $R^5$ Wasserstoff bedeuten.

Besonders bevorzugte Ammoniumionen entsprechend Formel 22 sind solche, bei denen unabhängig voneinander

$R^7$ für Acetyl, Methyl, Ethyl oder n-Butyl und

$R^1$, $R^2$, und $R^3$ für Methyl, Ethyl, n-Butyl, 2-Hydroxyethyl, Benzyl oder Phenyl stehen.

Besonders bevorzugte Phosphoniumionen entsprechend Formel 23 sind solche, bei denen unabhängig voneinander

$R^7$ für Acetyl, Methyl, Ethyl oder n-Butyl und

$R^1$, $R^2$, und $R^3$ für Phenyl, Phenoxy, Ethoxy und n-Butoxy

stehen.

Unter diesen sind die Ammonium-, Phosphonium-, Pyridinium- und Imidazoliumionen bevorzugt.

Ganz besonders bevorzugt sind als Kationen 1,2-Dimethylpyridinium, 1-Methyl-2-ethylpyridinium, 1-Methyl-2-ethyl-6-methylpyridinium, N-Methylpyridinium, 1-Butyl-2-methylpyridinium, 1-Butyl-2-ethylpyridinium, 1-Butyl-2-ethyl-6-methylpyridinium, n-Butylpyridinium, 1-Butyl-4-methylpyridinium, 1,3-Dimethylimidazolium, 1,2,3-Trimethylimidazolium, 1-n-Butyl-3-methylimidazolium, 1,3,4,5-Tetramethylimidazolium, 1,3,4-Trimethylimidazolium, 2,3- Dimethylimidazolium, 1-Butyl-2,3-dimethylimidazolium, 3,4-Dimethylimidazolium, 2-Ethyl-3,4-dimethylimidazolium, 3-Methyl-2-ethylimidazol, 3-Butyl-1-methylimidazolium, 3-Butyl-1-ethylimidazolium, 3-Butyl-1,2-dimethylimidazolium, 1,3-Di-n-Butylimidazolium, 3-Butyl-1,4,5- Trimetylimidazoliuin, 3-Butyl-1,4-Dimethylimidazolium, 3-Butyl-2-methylimidazolium, 1,3-Dibutyl-2-methylimidazolium, 3-Butyl-4-methylimidazolium, 3-Butyl-2-ethyl-4-methylimidazolium und 3-Butyl-2-ethyl-imidazolium, 1-Methyl-3-Octylimidazolium, 1-Decyl-3-Methylimidazolium.

Insbesondere bevorzugt sind 1-Butyl-4-methylpyridinium, 1-n-Butyl-3-methylimidazolium und 1-n-Butyl-3-ethylimidazolium.

Als Anionen sind prinzipiell alle Anionen denkbar.

Bevorzugt als Anionen sind Halogenide, $F^-$, $Cl^-$, $Br^-$, $I^-$, Acetat $CH_3COO^-$, Trifluoracetat $CF_3COO^-$, Triflat $CF_3SO_3^-$, Sulfat $SO_4^{2-}$, Hydrogensulfat $HSO_4^-$, Methylsulfat $CH_3OSO_3^-$, Ethylsulfat, $C_2H_5OSO_3^-$, Sulfit $SO_3^{2-}$, Hydrogensulfit $HSO_3^-$, Aluminiumchloride $AlCl_4^-$, $Al_2Cl_7^-$, $Al_3Cl_{10}^-$, Aluminiumtetrabromid $AlBr_4^-$, Nitrit $NO_2^-$, Nitrat $NO_3^-$, Kupferchlorid $CuCl_2^-$, Phosphat $PO_4^{3-}$, Hydrogenphosphat $HPO_4^{2-}$, Dihydrogenphosphat $H_2PO_4^-$, Carbonat $CO_3^{2-}$, Hydrogencarbonat $HCO_3^-$.

Besonders bevorzugt sind Tetrafluoroborat $BF_4^-$, Hexafluorophosphat $PF_6^-$, Bis(trifluormethylsulfonyl)imid $(CF_3SO_2)_2N^-$, Tosylat $p\text{-}CH_3C_6H_4SO_3^-$.

Unter den Ionischen Flüssigkeiten sind solche ganz besonders bevorzugt, deren Salze einen $E_T(30)$- Wert von > 20, bevorzugt von >30, besonders bevorzugt von > 40 aufweisen. Der $E_T(30)$-Wert ist ein Maß für die Polarität und wird von C. Reichardt in Reichardt, Christian Solvent Effects in Organic Chemistry Weinheim : VCH, 1979. - XI, (Monographs in Modern Chemistry ; 3), ISBN 3-527-25793-4 Seite 241 beschrieben.

Die erfindungsgemäßen Polymere können eine beliebige Polymerarchitektur besitzen, bevorzugt linear, verzeigt oder auch hyperverzweigt als Homopolymere oder als alternierende, statistische oder Gradienten-Copolymere, als Pfropf,- Kamm- oder Blockcopolymere, besonders bevorzugt hyperverzweigt als Homopolymere oder als alternierende, statistische oder Gradienten-Copolymere. Unter hyperverzweigten Polymeren werden solche verstanden, wie sie von Kim, Y. H. (in Journal of Polymer Science, Part A: Polymer Chemistry, 1998, 36, 1685) sowie Hult, A., Johannson, M., Malmström, E. (in Advances in Polymer Science, 1999, 143) und/oder Arlt et al. (in DE 10160518 A1) definiert sind. Die verwendeten Polymere haben bevorzugt eine Molmasse zwischen 800 g/mol und 500000 g/mol, besonders bevorzugt zwischen 1000 g/mol und 50000 g/mol und können als funktionelle Gruppen solche Gruppen, die im Lehrbuch der Organischen Chemie (H. Beyer und W. Walter; S. Hirzel Verlag Stuttgart, 21. Auflage; 1988) aufgeführt sind - bevorzugt OH-, Carbonyl-, Carboxyl-, Amino-, Mercapto-, oder Nitro-Gruppen - allein oder nebeneinander, aufweisen. Die verwendeten Polymere können u. a. aus den im Lehrbuch der Organischen Chemie (H. Beyer und W. Walter; S. Hirzel Verlag Stuttgart, 21. Auflage; 1988) und/oder DE10160518A1 aufgeführten Polymeren ausgwewählt sein - bevorzugt Polyether, aliphatische Polyester, Polyglycerine, Polyphenylene, Polypropylenimin, Polyamidoamin oder Poly(meth)acrylsäurederivate - und einzeln oder in Mischungen eingesetzt werden.

Polymermischungen oder Mischungen von einem oder mehreren Polymeren mit einer oder mehreren Ionischen Flüssigkeiten sind erfindungsgemäß verwendbar.

Denkbar sind auch Absorptionsmittel oder Emulsionen oder Suspensionen mit schwerflüchtigen Fluiden. Ferner wären auch fließfähige, z.B. pulver- oder granolenförmige Feststoffe, die z.B. oberflächig kontaminiert sind, behandelbar.

Bei den nach dem erfindungsgemäßen Verfahren abzutrennenden flüchtigen Fluiden handelt es sich um alle Fluide (insbesondere Flüssigkeiten oder verflüssigte Gase oder permanente Gase), die bei der betrachteten Destillation einen höheren Verteilungskoeffizienten $y_i/x_i$ aufweisen als das schwerflüchtige Fluid, von dem diese abgetrennt werden sollen.

Im erfindungsgemäßen Reinigungsverfahren abzutrennende flüchtige Komponenten können beispielsweise, aber nicht abschließend, sein:

- aromatische und aliphatische Kohlenwasserstoffe
- Alkane und Alkene mit einer Kohlenstoffzahl zwischen 1 und 12, bevorzugt zwischen 2 und 10
- Ketone
- Amide und Säuren, bevorzugt Carbonsäuren
- Alkohole, Acetate, Ether
- Sulfide
- Halogenkohlenwasserstoffe
- Furane
- Cyclische Verbindungen obiger Spezies
- Wasser

Im erfindungsgemäßen Verfahren verwendete Hilfsstoffe sind in einer bevorzugten Ausführung des Verfahrens chemisch weitgehend inert. Es sind - je nach Art der Anwendung - aber natürlich auch Hilfsstoffe möglich, die bei Einsatz/ Wiedereinsatz in einem assoziierten Trennverfahren zusätzlich die Aktivitätskoeffizienten der zu trennenden Fluide günstig beeinflussen und/oder bei Einsatz/Wiedereinsatz in einer assoziierten chemischen Reaktion eine Funktion übernehmen.

Bezüglich der chemischen Struktur der einzusetzenden Hilfsstoffe und zu trennenden Komponenten A und B gibt es keine generellen Einschränkungen sofern sie thermisch stabil genug sind und bzgl. der Partialdrücke und Dampfdrücke die hier an anderen Stellen geforderten Eigenschaften aufweisen. Bevorzugt werden solche chemischen Verbindungen, die aus Lehrbüchern und/oder Nachschlagewerken der Chemie und/oder Ingenieurwissenschaften bekannt sind,

u.a. sämtliche chemischen Verbindungen deren Namen oder Gruppe in einer oder mehreren der folgenden Literaturquellen genannt wird: "Lehrbuch der Organischen Chemie" ISBN 3777604380, "Lehrbuch der Organischen Chemie" ISBN 3527260676, "Lehrbuch der Anorganischen Chemie" ISBN 3110075113 sowie "Ullmann's Encyclopedia of Industrial Chemistry" ISSN 14356007, Beilstein, Gmelin, DE 10136614, EP 1372807 und DE 10160518. Als Hilfsstoff ist auch ein Stoff denkbar, der - z.B. durch thermische Zersetzung oder chemische Reaktion - erst innerhalb der Rektifikation die benötigten Eigenschaften entwickelt (z.B. ein schwerflüchtiger Hilfsstoff, der bei der Sumpftemperatur einen flüchtigeren Hilfsstoff freisetzt, der zu einem gewissen Sumpfdruck führt).

Unter Rektifikation wird erfindungsgemäß eine mehrstufige Destillation unter Rückfluss verstanden. Kernelemente einer Rektifikationskolonne sind der Verdampfer im Sumpf, mit dem ein aufsteigender Dampfstrom erzeugt wird, und der Kondensator am Kopf, mit dem ein absteigender Flüssigkeitsstrom erzeugt wird. Aufsteigender Dampfstrom und absteigender Flüssigkeitsstrom werden an Einbauten intensiv in Kontakt miteinander gebracht. Durch den so erzeugten Gegenstrom ist es bei Verwendung von Verstärkerteil und Abtriebsteil möglich, die Zusammensetzungen der Ströme am Kopf und Sumpf der Kolonne mit Hilfe der Parameter Kolonnenhöhe und Rücklaufverhältnis in weiten Bereichen einzustellen. Außer in einer üblichen Kolonne kann eine Rektifikation auch in geeignet verschalteten Apparaten und Maschinen durchgeführt werden. Als mehrstufig wird eine solche Rektifikation hier angesehen, wenn ein oder mehr Gleichgewichtsstufen und ein Partialkondensator vorliegen oder zwei oder mehr Gleichgewichtsstufen und ein Totalkondensator. Gleichgewichtsstufen können hierbei Teile einer Kolonne oder eines Behälters sein, die so betrieben werden könnten, dass ungefähr eine thermodynamische Gleichgewichtseinstellung möglich wäre.

Ebenfalls Gegenstand der Erfindung ist ein Trennverfahren, bei dem die ionischen Flüssigkeiten und/oder die Polymere als spezielle Zusatzstoffe verwendet werden, dadurch gekennzeichnet, dass die als spezielle Zusatzstoffe eingesetzten die ionischen Flüssigkeiten und/oder Polymere in einem oder mehreren assoziierten, d.h. vorangehenden, zwischengeschalteten und/oder nachfolgenden Prozessschritten durch das erfindungsgemäße Verfahren von flüchtigen Verunreinigungen getrennt werden.

Dabei sind als Trennverfahren solche bevorzugt, bei denen Fluide voneinander getrennt werden, besonders bevorzugt die Extraktivrektifikation oder die Flüssig-Flüssig Extraktion oder Membranverfahren, ganz besonders bevorzugt die Extraktivrektifikation. Unter Extraktiv-Rektifikation wird das Trennverfahren verstanden, wie es in [Stichlmair, S. und Fair, J., Distillation, ISBN 0-471-25241-7, Seite 241 ff] beschrieben ist. Unter "Entrainer" wird in dieser Patentschrift allerdings in Erweiterung des genannten eine ionische Flüssigkeit und/oder ein Polymer, das rein oder als Mischung vorliegen kann, verstanden.

Eine Flüssig-Flüssig- Extraktion wird in [Sattler, K., Thermische Trennverfahren, ISBN 3-527-28636-5, Kapitel 6] beschrieben. Unter "Solvent" wird in dieser Patentschrift allerdings in Erweiterung des genannten eine ionische Flüssigkeit und/oder ein Polymer, das rein oder als Mischung vorliegen kann, verstanden.

Membranverfahren, wie sie im Rahmen des erfindungsgemäßen Trennverfahrens zum Einsatz kommen können, werden z.B. in Membranverfahren, T. Melin und R. Rautenbach, Springer-Verlag, 2004 beschrieben.

In einer bevorzugten Variante des erfindungsgemäßen Verfahrens kann der Hilfsstoff zusammen mit dem gereinigten Polymer und/oder der gereinigten ionischen Flüssigkeit wieder in das kontinuierliche oder diskontinuierliche Trennverfahren eingebracht werden, bevorzugt wird hierbei eine kontinuierliche Rückführung der Komponenten in den laufenden Prozess. Besonders bevorzugt wird eine kontinuierliche Rückführung in kontinuierlich betriebenen Verfahren.

In einer besonders bevorzugten Ausführung des erfindungsgemäßen Verfahrens unterstützen die Hilfsstoffe ihrerseits zusätzlich die Trennung dadurch, dass sie eine Veränderung des Trennfaktors der zu trennenden Komponenten abweichend von 1 bewirken.

Im erfindungsgemäßen Trennverfahren zu trennende Gemische können beispielsweise, aber nicht abschließend, ausgewählt sein aus

- einer Mischung, die einen oder zwei oder mehr der weiter oben als "leichterflüchtig" bezeichneten Stoffe sowie einer beliebige Anzahl weiterer Stoffe enthält.:
- einer Mischung, die aromatische und aliphatische, auch cyclische Kohlenwasserstoffe enthält,
- einer Mischung, die Alkane und Alkene mit einerr Kohlenstoffzahl zwischen 3 und 12, bevorzugt zwischen 4 und 10 enthält,
- einer Mischung, die Ketone und alicyclische Verbindungen enthält,
- einer Mischung, die Amide und Säuren, bevorzugt Carbonsäuren, enthält,
- einer Mischung, die Alkohole und Alkane enthält,

- einer Mischung, die Alkohole und Aromaten enthält,
- einer Mischung, die Ketone und Alkohole enthält,
- einer Mischung, die Acetate und Ketone enthält,
- einer Mischung, die Ether und Alkane enthält,
- einer Mischung, die Ether und Alkene enthält,
- einer Mischung, die Sulfide und Ketone enthält,
- einer Mischung, die Halogenkohlenwasserstoffe und Ketone enthält,
- einer Mischung, die cyclische Ketone und cyclische Alkohole enthält,
- einem System, das neben Wasser Alkohole mit einer Kohlenstoffzahl zwischen 1 und 12, bevorzugt zwischen 1 und 8, besonders bevorzugt zwischen 1 und 5, organische Säuren, vorzugsweise Alkansäuren, Ketone, Furane enthält.

Ebenfalls Gegenstand der Erfindung sind chemische Reaktionen, bei denen mindestens eine ionische Flüssigkeit und/oder ein Polymer anwesend ist, dadurch gekennzeichnet, dass diese in mindestens einem assoziierten Prozessschritt durch das erfindungsgemäße Reinigungsverfahren von flüchtigen Komponenten befreit wird.

Unter chemischen Reaktionen werden solche verstanden wie sie beispielsweise, aber nicht abschließend, in "Ionic Liquids in Synthesis" (P. Wasserscheid und T. Welton, Wiley-VCH, ISBN 3-527-30515-7) beschrieben sind. In einer möglichen Ausführung der erfindungsgemäßen chemischen Reaktionen sind die verwendeten Hilfsstoffe chemisch weitgehend inert. In einer bevorzugten Ausführung der erfindungsgemäßen chemischen Reaktion kann der erfindungsgemäße Hilfsstoff eine weitere Funktion in der chemischen Reaktion wahrnehmen, beispielsweise als Edukt, Zwischenprodukt, Produkt, (Co)Solvenz, Lösevermittler und/oder Katalysator, bevorzugt als Edukt, Produkt, (Co)Solvenz oder Lösevermittler, besonders bevorzugt als Edukt, (Co)Solvenz oder Lösevermittler.

Ebenfalls Gegenstand der Erfindung sind Verfahren, bei denen mindestens Ionische Flüssigkeiten und/oder Polymere als Stabilisatoren eingesetzt werden und die dadurch gekennzeichnet sind, dass diese in mindestens einem assoziierten Prozessschritt durch das erfindungsgemäße Reinigungsverfahren von flüchtigen Komponenten befreit werden.

Außerdem sind Verfahren ebenfalls Gegenstand der Erfindung, bei denen Ionische Flüssigkeiten und/oder Polymere, als Schmiermittel eingesetzt werden und die dadurch gekennzeichnet sind, dass diese - in mindestens einem assoziierten Prozessschritt durch das erfindungsgemäße Reinigungsverfahren von flüchtigen Komponenten befreit werden.

Ebenfalls Gegenstand der Erfindung sind Produkte, die mit Hilfe der erfindungsgemäßen Trennungsverfahren, der erfindungsgemäßen chemischen Reaktionen und/oder der erfindungsgemäßen Verfahren zugänglich werden.

Die Erfindung wird nachstehend anhand des folgenden Beispiels näher erläutert ohne sie jedoch auf dieses zu beschränken.

Es zeigen:

Fig. 1 das Schema einer Extraktivdestillation zur Trennung des Gemisches A+B mit Reinigung und Rückführung des Entrainers "E".

Fig. 2 das Schema einer Extraktivdestillation zur Trennung des Gemisches A+B mit Reinigung und Rückführung des Entrainers E unter Zuhilfenahme des Hilfsstoffes "H", der ebenfalls rückgeführt wird.

Tabelle 1 die gemessenen Stoffdaten bzgl. der Ionischen Flüssigkeit (IL) [bmim][$PF_6$].

Tabelle 2 die Parameter für das NRTL-Modell in Aspen Plus®

Tabelle 3 die berechneten Ströme des beispielhaft beschriebenen Prozesses (aus Fig. 2).

Gleichung 1 zur Berechnungen von Aktivitätskoeffizienten nach dem NRTL-Aktivitätskoeffizientenmodell von H. Renon und J.M. Prausnitz (AIChE Journal 14, 1968, Seite 135) in der in Aspen Plus®:Version 11.1.1 verwendeten Form.

## Beispiel

Es wird ein Prozess wie in Fig. 2 schematisch dargestellt betrachtet, bei dem die Ionische Flüssigkeit 1-*n*-Butyl-3-Methylimidazoliumhexafluorphosphat [bmim][$PF_6$] als Entrainer in der Extraktivdestillation eingesetzt wird, um die Trennung des azeotropen Gemisches von Cyclohexan und Benzol in einer Destillationskolonne zu ermöglichen.

p-Dichlorbenzol (p-DCB) wird als Hilfsstoff in Sinne der Erfindung eingesetzt, um nach Abtrennung des Kopfproduktes Cyclohexan in der Extraktivrektifikationskolonne den zurückzuführenden Entrainer in einer zweiten Destillationskolonne mittels Rektifikation von Benzol zu befreien. Das p-DCB wird zusammen mit der Ionischen Flüssigkeit im Kreis gefahren, da die geringen Mengen p-DCB in der Extraktivdestillation nicht stören. Eine beliebig vollständige und industriell einsetzbare Abtrennung des Benzols aus dem schwerflüchtigen [bmim][$PF_6$] wird so erstmals destillativ möglich.

Als Prozessgrößen werden in diesem Beispiel gewählt:

Zu trennendes Gemisch: 0,9t/h Benzol + 0, 1t/h Cyclohexan,

Trennhilfsstoffe für Kolonne 1: 1,5t/h [bmim][$PF_6$], 0,1t/h p-Dichlorbenzol

Kolonne 1: 27 Stufen, Feedzugabe: 14. Stufe, Entrainerzugabe: 3. Stufe, 800-815 mbar, Rückfluss = 0,25t/h

Kolonne 2: 18 Stufen, Feedzugabe: 11. Stufe, 150-160 mbar, Rückfluss = 0,45t/h

Eine mit dem Prozesssimulator Aspen Plus® durchgeführte Simulationsrechnung liefert die Massenströme in dem Prozess. Die benötigten Eigenschaften der Ionischen Flüssigkeit wie die Dichte bei verschiedenen Temperaturen oder auch die Aktivitätskoeffizienten der zu trennenden Komponenten und des Hilfsstoffes in der Ionischen Flüssigkeit in unendlicher Verdünnung, die weder in der Aspen Plus® Datenbank noch in der zugänglichen Literatur vorhanden waren, wurden mittels eigener Messungen ermittelt und sind in Tabelle 1 tabelliert. Die Dichten wurden mittels Pyknometer bestimmt, die Aktivitätskoeffizienten mittels Inverser Gaschromatographie (analog A. Heintz, D. Kulikov, S. Verevkin in J. Chem. Eng. Data 2001, 46, 1526-1529 und den dortigen Quellenangaben).

Zur Simulation der Gleichgewichte in Aspen Plus® wurde das NRTL-Aktivitätskoeffizientenmodell (s.o.) verwendet, die Parameter sind in Tabelle 2 aufgeführt. Vereinfachend wird angenommen, dass die Dichte der Ionischen Flüssigkeit linear bzgl. der Temperatur ist und die Aktivitätskoeffizienten bei unendlicher Verdünnung in der Ionischen Flüssigkeit unabhängig von der Temperatur sind.

Tabelle 3 zeigt die Eigenschaften der Ströme dieses Prozesses: den Kopf der Extraktivdestillationskolonne (Kolonne 1) verlassen 99,74 % des eingesetzten Cyclohexans mit einer Reinheit von 99,77 Gew.-%. Dieser Strom enthält als Verunreinigungen nur 0,2 Gew. % Benzol und 0,03 Gew.% p-DCB. 99,98 % des eingesetzten Benzols werden am Kopf der Reinigungskolonne (Kolonne 2) wiedergewonnen - verunreinigt mit nur 0,03 Gew.% Cyclohexan. Die Ionische Flüssigkeit verlässt zusammen mit dem p-DCB die Reinigungskolonne am Sumpf und wird in die Extraktivdestillationskolonne zurückgeführt. Wegen des niedrigen Dampfdrucks von p-DCB reichen 3 Stufen oberhalb des Entrainerzulaufs, um die p-DCB-Verunreinigung im Cyclohexan auf 0,03 Gew. % zu halten. Die geringen Verluste am Hilfsstoff p-DCB, die dadurch entstehen, dass geringe Mengen hiervon in das Kopfprodukt von Kolonne 1 gelangen, werden dadurch ausgeglichen, dass der Entrainerzuführung von Kolonne 1 permanent 0,03 kg/h frisches p-DCB zugeführt werden.

**Tabelle 1**

| **Temperatur in °C** | 20 | 110 | 169,6 |
|---|---|---|---|
| **IL-Dichte in kg/m³** | 1376 | 1303 | 1253 |
| | | | |
| | **Cyclohexan** | **Benzol** | **p-Dichlorbenzol** |
| **Aktivitätskoeffizient in unendlicher Verdünnung in der IL bei 100°C** | 17,9 | 1,87 | 4,35 |

**Tabelle 2**

| Komp. i | Cyclohexan | P-DB | Benzol | Cyclohexan | Cyclohexan | p-DCB |
|---|---|---|---|---|---|---|
| Komp. j | Benzol | Benzol | IL | p-DCB | IL | IL |
| $a_{ij}$ | 0 | 0 | 40 | 0 | 4 | 40 |
| $a_{ji}$ | 0 | 0 | 0,57 | 0 | 1,6495 | 1,5504 |
| $b_{ij}$ | -43,3406 | 273,0688 | 0 | 0 | 0 | 0 |
| $b_{ji}$ | 182,7545 | -315,3037 | 0 | 0 | 0 | 0 |
| $\alpha_{ij}$ | 0,3 | 0,3 | 0,3 | 0,3 | 0,3 | 0,3 |

**Tabelle 3**

| | Kolonne 1 Feed | Kolonne 1 Entrainer | Kolonne 1 Kopfprodukt | Kolonne 1 Sumpfprodukt | Kolonne 2 Kopfprodukt |
|---|---|---|---|---|---|
| Strom in Abb. 2 | A+B | E+H | A | B+E+H | B |
| Temperatur in °C | 71,7 | 47,5 | 73,3 | 85,3 | 28,4 |
| Druck in bar | 0,82 | 1,2 | 0,8 | 0,815 | 0,15 |
| [bmim][$PF_6$] (E) in kg/h | 0 | 1500 | 0 | 1500 | 0 |
| p-DCB (H) in kg/h | 0 | 99,97 (*) | 0,03 | 99,94 | 0 |
| Cyclohexan (A) in kg/h | 100 | 0 | 99,74 | 0,26 | 0,26 |
| Benzol (B) in kg/h | 900 | 0,3 | 0,2 | 900,1 | 899,8 |
| (*) inkl. 0,03 kg/h frisches p-DCB. | | | | | |

**Gleichung 1**

$$\ln \gamma_i = \frac{\sum_j x_j \tau_{ji} G_{ji}}{\sum_k x_k G_{ki}} + \sum_j \frac{x_j G_{ij}}{\sum_k x_k G_{kj}} \left( \tau_{ij} - \frac{\sum_m x_m \tau_{mj} G_{mj}}{\sum_k x_k G_{kj}} \right)$$

wobei Gij = exp($-\alpha_{ij}\,\tau_{ij}$) und $\tau_{ij} = a_{ij} + \frac{b_{ij}}{T}$, $\alpha_{ij} = \alpha_{ji}$ und $\tau_{it} = 0$.

*T* ist die Temperatur in Kelvin

$x_i$ ist der Molenbruch der Komponente *i*

$\alpha_{ij}$, $\alpha_{ij}$ und $b_{ij}$ sind die anpassbaren Modellparameter

$\tau_{ij}$ und $G_{ij}$ sind Hilfsgrößen, die mit obigen Formeln definiert sind

$\gamma_i$ ist der Aktivitätskoeffizient der Komponente *i*

Durch Einschränkungen bei der praktischen Ausführung sind unwesentliche Abweichungen von den theoretischen Berechnungen möglich.

## Patentansprüche

1. Verfahren zur Abtrennung einer oder mehrerer flüchtiger Komponenten von ein oder mehreren schwerflüchtigen Fluiden, welche ionische Flüssigkeiten und/oder Polymere sind, durch mehrstufige Rektifikation, **dadurch gekennzeichnet, dass**

   (i) mindestens ein flüchtiger Hilfsstoff verwendet wird,
   (ii) der verwendete Hilfsstoff bzw. das Hilfsstoffgemisch im Sumpf der Destillationseinheit durch seinen Partialdruck einen Anteil von mindestens 50% zum Druck beisteuert und
   (iii) der Druck im Sumpf der Kolonne abzüglich des dort herrschenden Partialdrucks des Hilfsstoffes bzw. des Hilfsstoffgemisches 10 mbar nicht überschreitet,
   (iv) der verwendete Hilfsstoff bzw. das Hilfsstoffgemisch die Destillationseinheit größtenteils zusammen mit den schwerflüchtigen Fluiden am Sumpf verlässt, und
   (v) der mindestens eine Hilfsstoff einen Verteilungskoeffizient aufweist, der höher ist, als jener der ein oder mehreren schwerflüchtigen Fluide, und niedriger ist, als jener der ein oder mehreren flüchtigen Komponenten.

2. Verfahren gemäß Anspruch 1, **dadurch gekennzeichnet, dass** es sich bei dem schwerflüchtigen Fluid um mindestens eine Ionische Flüssigkeit handelt.

3. Verfahren gemäß Anspruch 1, **dadurch gekennzeichnet, dass** es sich bei dem schwerflüchtigen Fluid um mindestens ein Polymer handelt.

4. Verfahren nach einem der Ansprüche 1 bis 3, **dadurch gekennzeichnet, dass** das mindestens eine schwerflüchtige Fluid nachfolgend in einer Extraktivrektifikation als Entrainer benutzt wird.

5. Verfahren nach einem der Ansprüche 1 bis 3, **dadurch gekennzeichnet, dass** das mindestens eine schwerflüchtige Fluid nachfolgend in einer Flüssig-Flüssig-Extraktion als Solvent benutzt wird.

6. Verfahren nach einem der Ansprüche 1 bis 3, **dadurch gekennzeichnet, dass** das mindestens eine schwerflüchtige Fluid nachfolgend in einem Membranverfahren als Solvent benutzt wird.

7. Verfahren nach einem der Ansprüche 1 bis 3, **dadurch gekennzeichnet, dass** nachfolgend das mindestens eine schwerflüchtige Fluid einer chemischen Reaktion zugeführt wird, bei der das schwerflüchtige Fluid anwesend ist.

8. Verfahren gemäß einem der Ansprüche 4-7, **dadurch gekennzeichnet, dass** der Hilfsstoff mit dem zu reinigenden schwerflüchtigen Fluid zusammen wieder eingesetzt werden kann und/oder weitgehend im Kreis gefahren wird, ohne dass der Hilfsstoff entfernt werden muss.

## Claims

1. Method for separating one or more volatile components from one or more sparingly volatile fluids which are ionic liquids and/or polymers by multistage rectification, **characterized in that**

   (i) at least one volatile auxiliary is used,
   (ii) the auxiliary used or the auxiliary mixture in the bottom of the distillation unit contributes a proportion of at least 50% to the pressure owing to its partial pressure and
   (iii) the pressure in the bottom of the column minus the partial pressure of the auxiliary or of the auxiliary mixture which prevails there does not exceed 10 mbar,
   (iv) the auxiliary used or the auxiliary mixture leaves the distillation unit for the most part together with the sparingly volatile fluids at the bottom, and
   (v) the at least one auxiliary has a partition coefficient higher than that of the one or more sparingly volatile fluids, and lower than that of the one or more volatile components.

2. Method according to Claim 1, **characterized in that** the sparingly volatile fluid is at least one ionic liquid.

3. Method according to Claim 1, **characterized in that** the sparingly volatile fluid is at least one polymer.

4. Method according to any of Claims 1 to 3, **characterized in that** the at least one sparingly volatile fluid is subsequently used as entrainer in an extractive rectification.

5. Method according to any of Claims 1 to 3, **characterized in that** the at least one sparingly volatile fluid is subsequently used as solvent in a liquid-liquid extraction.

6. Method according to any of Claims 1 to 3, **characterized in that** the at least one sparingly volatile fluid is subsequently used as solvent in a membrane process.

7. Method according to any of Claims 1 to 3, **characterized in that** the at least one sparingly volatile fluid is subsequently supplied to a chemical reaction in which the sparingly volatile fluid is present.

8. Method according to any of Claims 4-7, **characterized in that** the auxiliary can be reused together with the sparingly volatile fluid to be purified and/or is substantially circulated without it being necessary to remove the auxiliary.

## Revendications

1. Procédé de séparation d'un ou de plusieurs composants volatils d'un ou de plusieurs fluides peu volatils, qui sont des liquides ioniques et/ou des polymères, par rectification à plusieurs étapes, **caractérisé en ce que**

   (i) au moins un adjuvant volatil est utilisé,
   (ii) l'adjuvant ou mélange d'adjuvants utilisé contribue à la pression au fond de l'unité de distillation par sa pression partielle en une proportion d'au moins 50 % et
   (iii) la pression au fond de la colonne, déduction faite de la pression partielle de l'adjuvant ou mélange d'adjuvants qui y règne, ne dépasse pas 10 mbar,
   (iv) l'adjuvant ou mélange d'adjuvants utilisé quitte l'unité de distillation en majeure partie avec les fluides peu volatils au fond, et
   (v) le ou les adjuvants présentent un coefficient de partage qui est supérieur à celui du ou des fluides peu volatils et inférieur à celui du ou des composants volatils.

2. Procédé selon la revendication 1, **caractérisé en ce que** le fluide peu volatil est au moins un liquide ionique.

3. Procédé selon la revendication 1, **caractérisé en ce que** le fluide peu volatil est au moins un polymère.

4. Procédé selon l'une quelconque des revendications 1 à 3, **caractérisé en ce que** le ou les fluides peu volatils sont ensuite utilisés dans une rectification extractive en tant qu'agent d'entraînement.

5. Procédé selon l'une quelconque des revendications 1 à 3, **caractérisé en ce que** le ou les fluides peu volatils sont ensuite utilisés dans une extraction liquide-liquide en tant que solvant.

6. Procédé selon l'une quelconque des revendications 1 à 3, **caractérisé en ce que** le ou les fluides peu volatils sont ensuite utilisés dans un procédé sur membrane en tant que solvant.

7. Procédé selon l'une quelconque des revendications 1 à 3, **caractérisé en ce que** le ou les fluides peu volatils sont ensuite introduits dans une réaction chimique lors de laquelle le fluide peu volatil est présent.

8. Procédé selon l'une quelconque des revendications 4 à 7, **caractérisé en ce que** l'adjuvant peut être réutilisé avec le fluide peu volatil à purifier et/ou est recyclé dans une large mesure sans que l'adjuvant ne doive être séparé.

Fig. 1

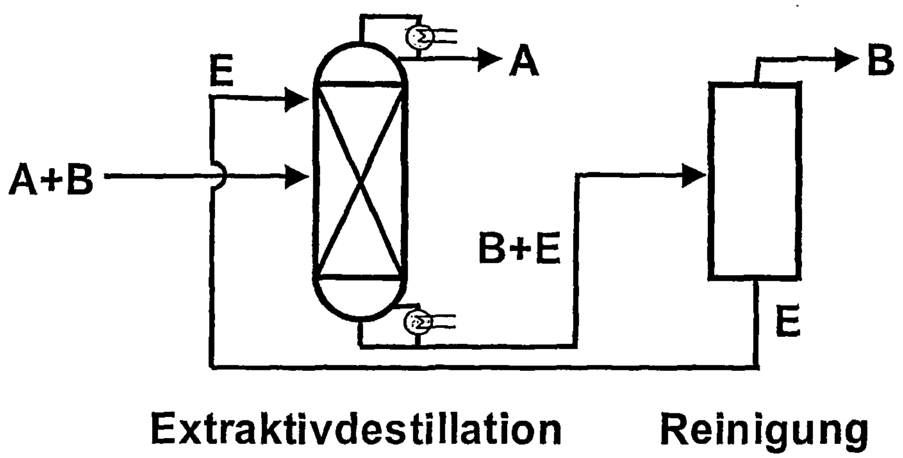
E
A
B
A+B
B+E
E
Extraktivdestillation
Reinigung

Fig. 2

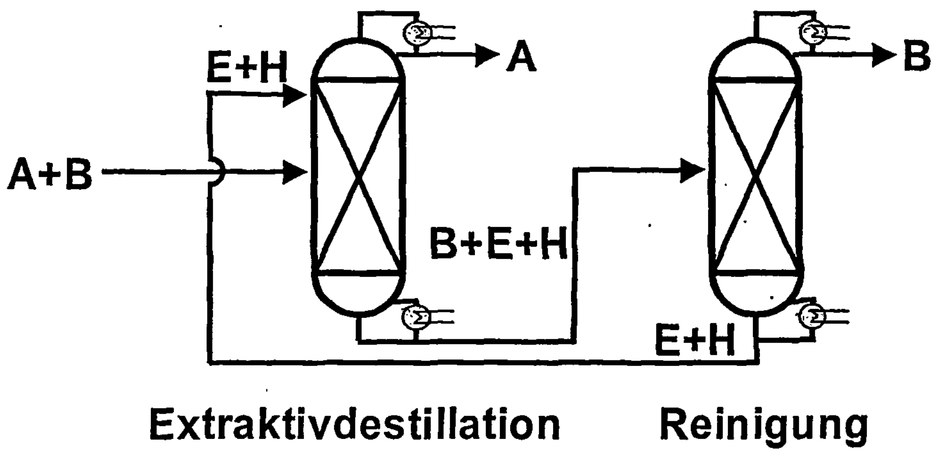
E+H
A
B
A+B
B+E+H
E+H
Extraktivdestillation
Reinigung

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 10136614
- EP 1372807 A
- DE 10160518
- DE 10136614 A1
- DE 10160518 A1
- EP 1372807 B1
- WO 9941752 A
- US 20030085156 A
- WO 200115175 A
- DE 10154052 A1
- DE 10206808 A1
- WO 03037835 A3
- WO 2002074718 A
- US 6339182 B1
- US 4359596 A
- US 5220106 A
- DE 19901524 A1
- WO 0016902 A
- WO 0198239 A1
- WO 03051894 A1
- WO 03062251 A1
- DE 19919494 A1


### In der Beschreibung aufgeführte Nicht-Patentliteratur


- **STICHLMAIR, S. ; FAIR, J.** *Distillation,* 1996, ISBN 0-471-25241-7, 241ff
- **GMEHLING ; BREHM.** Grundoperationen - Lehrbuch der Technischen Chemie. Thieme Verlag, 1996, vol. 2
- **SATTLER, K.** Thermische Trennverfahren
- **P. WASSERSCHEID ; T. WELTON.** Ionic Liquids in Synthesis. Wiley-VCH
- **T. MELIN ; R. RAUTENBACH.** Membranverfahren. Springer-Verlag, 2004
- Chemical & Engineering News. American Chemical Society, 29. April 2002
- **VON PROF. ALBRECHT SALZER.** *Chemical & Engineering News,* 29. April 2002, 4
- **P. WASSERSCHEID ; W. KEIM.** *Angewandten Chemie,* 2000, vol. 112, 3926-3945
- *Ionic Liquids in Synthesis,* ISBN 3527305157
- *Molten Salt Techniques,* ISBN 0306435543
- *Ionic Liquids,* ISBN 0841237891
- *Ionic Liquids as Green Solvents,* ISBN 0841238561
- *Green Industrial Applications of Ionic Liquids,* ISBN 1402011377
- *Chem. Rev.,* 1999, vol. 99, 2071-2083
- Monographs in Modern Chemistry. **C. REICHARDT.** Solvent Effects in Organic Chemistry. VCH, 1979, vol. XI, 241
- **KIM, Y. H.** *Journal of Polymer Science, Part A: Polymer Chemistry,* 1998, vol. 36, 1685
- **HULT, A. ; JOHANNSON, M. ; MALMSTRÖM, E.** *Advances in Polymer Science,* 1999, 143
- **H. BEYER ; W. WALTER ; S. HIRZEL.** Organischen Chemie. Verlag, 1988
- Lehrbuch der Organischen Chemie
- Lehrbuch der Anorganischen Chemie
- Ullmann's Encyclopedia of Industrial Chemistry
- **STICHLMAIR, S. ; FAIR, J.** *Distillation,* ISBN 0-471-25241-7, 241 ff
- **H. RENON ; J.M. PRAUSNITZ.** *AIChE Journal,* 1968, vol. 14, 135
- **A. HEINTZ ; D. KULIKOV ; S. VEREVKIN.** *J. Chem. Eng. Data,* 2001, vol. 46, 1526-1529